# EUROPÄISCHE PATENTSCHRIFT

(11) **EP 1 641 735 B1**
(45) Veröffentlichungstag und Bekanntmachung des Hinweises auf die Patenterteilung: **24.09.2008**
(21) Anmeldenummer: 04738847.5
(22) Anmeldetag: 02.07.2004
(51) Int. Cl.: C07C 51/41

(54) **VERFAHREN ZUR HERSTELLUNG VON METTALLSALZEN KURZKETTIGER, UNGESÄTTIGTER CARBONSÄUREN UND VERWENDUNG**
METHOD FOR THE PRODUCTION OF METAL SALTS OF SHORT-CHAINED, UNSATURATED, CARBOXYLIC ACIDS AND USE THEREOF
PROCEDE POUR PRODUIRE DES SELS METALLIQUES D'ACIDES CARBOXYLIQUES INSATURES A CHAINES COURTES ET UTILISATION DE CES SELS METALLIQUES

(30) Priorität: 03.07.2003 DE 10330217
(43) Veröffentlichungstag der Anmeldung: 05.04.2006
(73) Patentinhaber: Sasol Solvents Germany GmbH, 20537 Hamburg (DE)
(72) Erfinder: FINMANS, Peter, 47199 Duisburg (DE); HOELL, Detlef, 47441 Moers (DE); NICKEL, Eveline, 47495 Rheinberg (DE); GRAMSE, Elmar, 47445 Moers (DE)
(74) Vertreter: Schupfner, Georg
(86) Internationale Anmeldenummer: PCT/DE2004/001414
(87) Internationale Veröffentlichungsnummer: WO 2005/005364

(56) Entgegenhaltungen:
- BE-A- 763 157
- FR-A- 1 205 366
- US-A- 2 940 957
- US-A- 3 337 391
- US-A- 3 923 716
- US-A- 5 952 151
- US-A- 5 998 646
- PATENT ABSTRACTS OF JAPAN Bd. 0161, Nr. 52 (C-0929), 15. April 1992 (1992-04-15) & JP 4 008298 A (EIKEN CHEM CO LTD), 13. Januar 1992 (1992-01-13)
- DATABASE WPI Section Ch, Week 198847 Derwent Publications Ltd., London, GB; Class A18, AN 1988-334152 XP002313174 & JP 63 247037 A (DYNIC CORP) 13. Oktober 1988 (1988-10-13)
- DATABASE WPI Section Ch, Week 197920 Derwent Publications Ltd., London, GB; Class E12, AN 1979-38363B XP002313175 & SU 614 089 A (KOVYRZINA K A) 14. Juni 1978 (1978-06-14)

## Beschreibung

Gegenstand der Erfindung ist ein Verfahren zur Herstellung von Metallsalzen kurzkettiger, ungesättigter Carbonsäuren durch Umsetzung ungesättigter Carbonsäuren mit Metallalkoholaten. Gegenstand der Erfindung ist weiterhin die Verwendung von Metallsalzen kurzkettiger, ungesättigter Carbonsäuren.

Einfach ungesättigte Carbonsäuren haben in ihrer homologen Reihe die allgemeine Summenformel CₙH₂ₙ₋₁-COOH. Sie sind farblose Flüssigkeiten, die bei niedrigen Kettenlängen mit Wasser in jedem beliebigen Verhältnis mischbar sind und die Neigung haben, zu einer glasartigen Masse zu polymerisieren. Auch die Metallsalze der Acrylsäure sind farblos. Sie lassen sich in Lösung und als Pulver herstellen.

Verfahren zur Herstellung von Aluminiumsalzen der Acrylsäure sind an sich bekannt. So beschreibt die JP 48091012 die Herstellung von Aluminumacrylat und seiner basischen (oder komplexen) Salze durch Reaktion von Al(OH)₃ -Gel oder basischem Aluminiumsulfat, das aus einer wässrigen Lösung von Al₂(SO₄)₃ durch Entfernung des gesamten oder eines Teils des SO₄²⁻ als unlösliches Salz gewonnen wird, mit reiner Acrylsäure oder mit einem Gemisch aus Acrylsäure und einer organischen oder anorganischen Säure. Der Nachteil vorgenannten Verfahrens liegt in dem Anfall von Salzen, die entweder das Produkt verunreinigen oder in einem aufwendigen Separationsschritt abgetrennt werden müssen.

Auch die US 3,957,598 offenbart die Herstellung von Carbonsäure - Metallsalzen. Hiernach wird ein Metallsalz einer Carbonsäure gebildet, indem eine Carbonsäure mit einem aktivierten Metall zur Reaktion gebracht wird. Das aktivierte Metall bildet sich, wenn ein erstes Metall mit einem zweiten Metall mit Wasserstoffaffinität in Gegenwart einer Protonenquelle umgesetzt wird. Hierbei wird aktiviertes Aluminium aus einem hochaufgereinigtem Aluminiumstab eingesetzt und mit einer Legierung aus Gallium und Indium in Anwesenheit von Salzsäure umgesetzt. Durch Zufügen von Überschusswasser bildet sich in Anwesenheit von Carbonsäure und aktiviertem Metall ein polymeres Metallsalz aus einer wasserlöslichen Carbonsäure.

Nachteilig an diesem Verfahren ist die Notwendigkeit zur Nutzung legierten Aluminiummetalls, wobei die Komponenten der Legierung teilweise hoch umweltbedenklich sind und deren Rückgewinnung sehr aufwendige Separationstechniken erfordert.

Die US 3,923,716 beschreibt die Herstellung eines Aluminiumacrylats in zwei Schritten. Zunächst wird Acrylsäure zu wässriger Natronlauge gegeben, um Natriumacrylat zu erhalten, welches wiederum mit Aluminiumtrichlorid zu (Mono-, di-, tri-) Aluminiumacrylat und Natriumchlorid umgesetzt wird. Das Aluminiumacrylat kann aufgrund seiner geringen Löslichkeit aus dem wässrigen Reaktionsgemisch vom gelöstem Natriumchlorid abgetrennt werden. Nachteilig an diesem Verfahren ist, dass eine große Menge in Wasser gelösten und teilweise mit Produkt verunreinigten Kochsalzes entsteht und entsorgt werden muss.

Aus der US 5 998 646 ist die Herstellung entsprechende Salze des Titans bekannt. Die stetige Zufuhr von Sauerstoff zur Umsetzung und eine gewissen O₂-Sättigung wird in dieser aber nicht offenbart. Im Gegenteil wird unter reduziertem Druck bei Atmosphärenabschluss und erhöhter Temperatur umgesetzt, so dass die Abwesenheit von Sauerstoff und eine Lösungsmittelatmosphäre zu erwarten sind.

Aufgabe der vorliegenden Erfindung ist es, die vorbeschriebenen Nachteile zu überwinden, und insbesondere ein Verfahren bereit zustellen, bei dem ausschließlich unbedenkliche und leicht und vollständig abzutrennende Nebenprodukte entstehen und das zur Erzeugung sehr reiner Carbonsäuremetallsalze verschiedener Metallionen vorzugsweise unter Vermeidung des Anfalls ungewünschter polymerer Verbindungen geeignet ist, und ohne den Einsatz aufwendiger zusätzlicher Reinigungsschritte durchgeführt werden kann.

Die Aufgabe wird erfindungsgemäß gelöst durch ein Verfahren zur Herstellung von Metallsalzen kurzkettiger, ungesättigter Carbonsäuren durch Umsetzung
- von Metallalkoholat-Verbindungen
- mit Carbonsäuren der allgemeine Formel

   CₙH₂ₙ₋₁C(=O)OH

   mit Doppelbindung in 2- oder 3- Stellung, worin n für 2, 3, 4, 5 oder 6 steht und/oder Maleinsäure (weniger bevorzugt),
in Gegenwart von Sauerstoff, wobei man Sauerstoff stetig so zuführt, dass die Reaktionslösung zumindest zu 50% sauerstoffgesättigt ist, und die Metallsalze zumindest eine Gruppe der Formel

CₙH₂ₙ₋₁C(=O)O- und/oder -OC(=O)CH=CHC(=O)O-(H)

und folgende Metalle oder deren Mischungen

Al, Si, Sn, La, Zr, Cu und/oder Zn

aufweisen

Insbesondere weisen die Metallsalze kurzkettiger, ungesättigter Carbonsäuren die allgemeine Formel

M(OOCCₙH₂ₙ₋₁)ₐ(R¹)_{b}

auf und sind erhältlich durch Umsetzung einer linearen oder verzweigten, ungesättigten Carbonsäure der Formel

CₙH₂ₙ₋₁-COOH,

wobei n gleich 2, 3, 4, 5 oder 6 ist mit Doppelbindung in 2- oder 3- Stellung, vorzugsweise in 2-Stellung, mit einer Metallverbindung der allgemeinen Formel III

M(R¹)_{c}

und ggf. unter anderem

H(R¹) ,

worin
- a: zumindest 1 ist,
- b: 0, 1, 2 oder 3 ist und
- (a+b) und c: unabhängig voneinander eine ganze Zahl von 2 bis 4 sind,
- M: Al, Si, Sn, La, Zr, Cu oder Zn, insbesondere Al oder Zr ist,
- R¹: für eine Alkoholat-Gruppe mit einem C1- bis C6- Kohlenwasserstoffrest steht, wobei R¹ eine gesättigte, lineare oder verzweigte Alkoholat-Gruppe ist, herstellbar aus einem Alkohol mit zumindest einer -OH Gruppe, wobei die -OH Gruppen vorzugsweise primäre und/oder sekundäre -OH Gruppen sind, oder 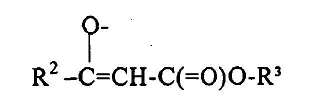 mit R² bzw. R³ gleich -CH₃, -C₂H₅, -C₃H₇ oder -C₄H₉ und
wobei n, R¹ , R² und R³ für jedes a, b bzw. c unterschiedlich sein können und zumindest ein R¹ in M(R¹)_{c} für eine Alkoholat-Gruppe mit einem C1- bis C6- Kohlenwasserstoffrest steht in Gegenwart von Sauerstoff (O₂), wobei man Sauerstoff stetig so zuführt, dass die Reaktionslösung zumindest zu 50% sauerstoffgesättigt ist.

Bevorzugte Ausführungsformen der Erfindung sind Gegenstand des weiteren unabhängigen Verfahrensanspruchs 2, der Unteransprüche oder nachfolgend erläutert.

Erfindungsgemäß wird die Umsetzung so in Gegenwart von Sauerstoff durchgeführt, dass die Reaktionslösung zumindest zu 50%, vorzugsweise zu zumindest 90%, sauerstoffgesättigt ist, etwa durch Zuführen eines Gasgemisches, das Sauerstoff in einer Konzentration von 5 bis 30 Vol.-%, vorzugsweise 15 bis 25 Vol.-% enthält. Geeignet ist auch die Zufuhr von Sauerstoff in Form eines Luftgemisches, insbesondere wenn dies getrocknet ist. Durch die Anwesenheit von Sauerstoff wird die Polymerisationsneigung des Produktes bei der Herstellung unterdrückt.

Das Verfahren erfolgt erfindungsgemäß bei Temperaturen von 0 °C bis 150 °C, vorzugsweise 20 bis 100 °C, besonders bevorzugt zumindest zeitweise bei über 40°C. Dabei wird erfindungsgemäß bevorzugt bei Drücken von 2 bar_{abs} bis 0,01 bar_{abs} gearbeitet.

Das Verfahren kann lösemittelfrei, d.h. abgesehen von obigen Reaktanten, ggf. im Überschuss, ohne zusätzliche Löse- oder Verdünnungsmittel durchgeführt werden. Soweit Lösemitteln eingesetzt werden sind geeignet Kohlenwasserstoffe, Ester, insbesondere Ester von C1- bis C18- Monocarbonsäuren mit C1- bis C8- Alkoholen und Etheralkoholen einschließlich Polyolen, Ether, Glycole und/oder Glycolmono-/di-ether.

Besonders bevorzugte ungesättigte Carbonsäuren im Sinne der Erfindung sind Acrylsäure und Methacrylsäure oder Carbonsäuren der Formel R-CH=CH-CH₂-COOH.

Die umgesetzten Metallverbindungen sind erfindungsgemäß Metallalkoholate. Das gebundene Metallion ist ein Ion der Metalle Magnesium, Calcium, Aluminium, Silizium, Zinn, Lanthan, Titan, Zirkon, Kupfer und Zink, bevorzugt Aluminium, Zirkonium und Titan, insbesondere Aluminium. Beispiele für erfindungsgemäße Metallalkoholate sind Aluminium-tri-*sek*.-butanolat, Aluminiumtriisopropylat, Zirkoniumtetrabutylat, Titantetrabutylat, Magnesiumdibutylat. Besonders bevorzugte Metallalkoholate sind Alkoholate von Alkoholen mit 3 bis 4 Kohlenstoffatomen, wie Isopropanol, n-Butanol und sek. Butanol, insbesondere Isopropanol und sek. Butanol. Gff. kann die Alkoholgruppe auch aus einem Polyol erhalten worden sein.

Wesentliche Vorteile des erfindungsgemäßen Verfahrens liegen in der sehr einfachen Herstellung eines hochreinen Produktes, das in reiner Form oder als Lösung in obigen Lösungsmitteln gewonnen bzw. aufgenommen werden kann. Das neuartige Verfahren ermöglicht einen Zugang zu Carbonsäuresalzen von Metallen, die in Form von Alkoholaten zur Verfügung gestellt werden können.

Im Vergleich zu den herkömmlichen Verfahren liefert das Verfahren u.a. folgende Verbesserungen:
- einfacherer Syntheseweg,
- geringe Polymerisationsneigung,
- höhere Reinheit in Bezug auf Fremdionen, vorzugsweise kleiner 100 ppm Chlorid und Sulfat,
- übertragbar auf Metalle, die als Alkoholate zur Verfügung stehen und,
- Produkt kann in hochreiner Form als Feststoff oder in Lösung hergestellt werden.

Die häufigsten Anwendungsbereiche der erfindungsgemäßen Metallverbindungen ungesättigter Carbonsäuren, insbesondere der Aluminium-mono bis tri-(Meth)Acrylate sind die Herstellung von Gummi-Werkstoffen, von synthetischen Harzen sowie von flammhemmenden Stoffen sowie für bzw. in Beschichtungen und Additiven, welche in Beschichtungen Anwendung finden. Die Beschichtungen können auf Gläsern, Keramiken, organischen Polymeren, Metallen, Papieren und Pappen erfolgen.

Weitere Anwendungsgebiete der Metall-(Meth)Acrylate sind:
Korrosionsschutz von Metallen, Beschichtung oder Herstellung von Glasfasern, Sandkernen, Papier, Kunststoffen etc., Ummantelung von (Gleichstrom)leitungen, Additive zu Baustoffen und lichthärtenden Zementen, lichtempfindliche und aufgrund von Lichteinwirkung bilderzeugende Medien sowie Photopapierbeschichtungen, Polituren, Stabilisatoren für Polymere, Entfernung feststofflicher Farben auf Wasserbasis, rheologieverbesserndes und als Fungizid wirksames Additiv und Trockenmittel bei der Herstellung von Lacken, Farben und Druckfarben sowie in der Medizin als Basis für Gelpflaster oder Zemente in der Zahnmedizin.

Metallsalze kurzkettiger, ungesättigter Carbonsäuren können Verwendung finden in Schutzbeschichtungen unterschiedlicher Natur. Als solche seien genannt Harze, wie Alkyd-, Epoxid- oder acryl-haltige Polymere, welche zur Versiegelung von Arbeitsplatten aus Marmor oder Naturstein, Parkett oder Faserplatten dienen können. Durch das Harz bekommt die Arbeitsplatte eine hohe Resistenz gegenüber aggressiven und/oder korrosiven Chemikalien, sowie gegenüber mechanischen und thermischen Beanspruchungen. Zum Einsatz im Stahlwasserbau, im Offshore-Bereich und im Schiffsbau werden Epoxidharze mit Bitumen oder Asphalt zu Epoxidharz/Teer-Beschichtungen kombiniert. Polyurethanbeschichtungen kommen im Möbel, Automobil und Flugzeugbau zum Einsatz. Ein weiteres Anwendungsgebiet ist die Schutzbeschichtung von Betonoberflächen, Marmorstatuen und ähnlichen Steinskulpturen. Phenolharze weisen eine hohe Korrosionsbeständigkeit auf und werden so sehr häufig in der Lebensmittelverpackung für Dosen genutzt. Modifizierte Phenolharze werden als Antifouling-Anstriche für maritime Anwendungen und als Basis für Korrosionsschutz für See- und Landfahrzeuge genutzt.

Der Zusatz von Metallsalzen kurzkettiger, ungesättigter Carbonsäuren zu diesen Harzen verbessert z.B. die Härte, gleichzeitig resultiert ein verbesserter Schutz gegen mechanische, thermische und chemische Einflüsse.

Weitere Beschichtungen, in denen Metallsalze kurzkettiger, ungesättigter Carbonsäuren erfindungsgemäß eingesetzt werden können, sind Lacke, lösemittel- und/oder wasserhaltig, z. B. Alkydharz-, PU-, NC-, Epoxid- und Acrylharzlacke. Derartige Lacke kommen zum Einsatz, um Werkstoffe, wie Metalle, Kunststoffe, Papiere oder Pappen und Hölzer zu versiegeln, und um diese somit vor Zersetzung oder Angriff durch aggressive Umweltbedingungen zu schützen. Als Beispiele seien ein Rostschutzlack auf einem Metalltreppengeländer einer Außentreppe oder der Schutzanstrich für Schiffe genannt. In analoger Weise wirken UV-Schutzlacke für Gartenmöbel, Holzschutzlacke oder imprägnierende Lacke für Papier oder Pappen. Acrylharzlacke werden erstens aufgrund ihrer schnellen Trocknung zur Lackierung von Kunststoffen (Phono/Fernsehgehäuse, Spielwaren, Kfz-Innenteile) und zweitens als chemisch vernetzende Lacke für hochwertige nichtvergilbende wetterbeständige Beschichtungen (2K-Lacke, Fassadenlacke, Einbrennlacke) verwendet. Lufttrocknende Alkydharzelacke werden prinzipiell für Bautenlacke, aber auch für Schiffs u. Maschinenfarben, Stahlkonstruktionen und für die Lackierung von Großraumfahrzeugen eingesetzt. Die Verwendungsmöglichkeiten für Beschichtungsstoffe auf Basis von Epoxidharzen, die bei Raumtemperatur härten, sind sehr vielfältige. Sie eignen sich für hochwertige säure- und lösemittelbeständige Beschichtungen in solchen Bereichen, die einer Einbrennlackierung nicht zugänglich sind, wie Rohre, Großbehälter, Boots- und Schiffskörper bis hin zum schweren Korrosionsschutz. In der industriellen Möbellackierung finden Nitrocellulose-Lacke ihren Einsatz, weil sie transparent und deckend, sowohl im Grund-, als auch im Decklack eingesetzt werden können. Nitrocellulose-Lacke liefern das beste Porenbild und bringen vor allem in offenporigen Lackierungen wie kein anderes Material den Holzcharakter zur Geltung. Die Lackierungen sind voll beständig gegen Feuchtigkeit und ausreichend gegen Alkohol, nicht aber gegen organische Lösungsmittel. Für Gravur und Offset-Druckfarben haben modifizierte Phenolharze als Binder große Bedeutung. Die Lösemittelbeständigkeit und Barriereeigenschaften dieser Lacke können durch den Zusatz von Metallacrylaten verbessert werden.

Die Metallsalze kurzkettiger, ungesättigter Carbonsäuren können auch in Polymerfolien aus PP oder PET, die z.B. als Verpackungsmaterial um den zu schützenden Gegenstand gelegt, aufgeklebt oder verschweißt werden, eingesetzt werden. Als Beispiel dafür seien Schutzfolien für Handydisplays oder Lebensmittel genannt. Glasgeräte, die hohen Drücken ausgesetzt sind und Verbundglasscheiben, werden ebenfalls durch Sicherheitsfolien vor dem Zerbersten geschützt.

In den beschriebenen Anwendungen sind durch Zusatz von Metallsalzen kurzkettiger, ungesättigter Carbonsäuren Vorteile im Hinblick auf Barriereeigenschaften, Kratzfestigkeit, Haftung und mech. Reißfestigkeiten erzielbar.

Metallsalze kurzkettiger, ungesättigter Carbonsäuren können neben den organischen Coatings auch in anorganischen Schutzbeschichtungen zum Einsatz kommen: z. B. geschieht dies durch Aufdampfen einer Metallschicht auf einen Gegenstand.

Eine weitere Anwendung solcher anorganischer Beschichtungen ist das Ausbilden einer Metalloxidschicht auf Metallen oder das Aufbringen einer solchen Metalloxidschicht auf einen Gebrauchsgegenstand, der z.B. metallische oder keramischen Oberflächen (auch Glas) aufweisen kann. Aluminium wird z.B. zum Schutz mit einer künstlichen Oxidschicht überzogen. Dieses Verfahren nennt man Eloxieren, oder allgemein: Anodisieren.

An dieser Stelle seien z.B. Diffusionssperren für den Lebensmittelbereich genannt. Ein Beispiel dieser Diffusionssperre ist die folgende Beschichtung: Sie besteht aus einer Aluminiumoxidschicht, die zwischen zwei Acrylsäurepolymerschichten aufgebracht wird. Diese Beschichtung soll den Sauerstofftransfer zwischen dem eingeschlossenem Produkt und der Umgebung minimieren.

Aluminiumoxid wird in vielen der oben genannten Anwendungen eingesetzt. Der Nachteil des Aluminiumoxides ist, dass es fest, unlöslich und wenig reaktiv ist. Es ist i.d.R. in organischen Verbindungen nicht löslich, so dass man spezielle Apparaturen oder Umsetzungen benötigt um diese dennoch darin einzuarbeiten.

Ziel der vorliegenden Erfindung ist die vorteilhafte Anwendung von Metallsalzen ungesättigter Carbonsäuren, die sich in Substanzen oder in Verbindung mit anderen Materialien allein oder in Mischungen, in flüssiger (gelöster) oder fester Form vielseitig verwenden lassen, mit ihnen mischbar / verträglich sind und über ihre reaktiven Gruppen polymerisierbar bzw. einbaubar sind, indem man die Vorteile von kurzkettigen, ungesättigten Carbonsäuren mit denen von Metalloxiden und /oder Metallsalzen vereint.

Die Kombination von polymerisierbaren organischen Gruppen und ionischen gebundenen Metallen eröffnet Wege zu Materialien, die flexibel einsetzbar sind und hohe Härten aufweisen. Darüber hinaus bieten die Wechselwirkungen zwischen LewisSäuren und -Basen weitere Möglichkeiten zur Festigkeitssteigerung neuer Materialien, bestehend aus den beschriebenen Lewis- oder nach Hydrolyse Brönstedt-Säuren und entsprechenden basischen Komponenten.

Die Vorteile polymerisierter Verbindungen liegen in einer erhöhten Umweltverträglichkeit und verringerter Geruchsbelästigung.

Überraschenderweise wurde gefunden, dass die organischen Metallsalze mit zumindest einer kurzkettigen (3 bis 7 Kohlenstoffatome), ungesättigten Carboxylgruppe für nachfolgende Verwendungen besonders gut geeignet sind:

Die beschriebenen Metall-organischen Verbindungen können hervorragend in der Oberflächenbeschichtung eingesetzt werden. Neben einer überraschend hohen Härte, verbunden mit guter Scheuerfestigkeit und Kratzbeständigkeit zeigen diese UV- oder Elektronenstrahl oder durch herkömmliche chemische Radikalstarter gehärteten Beschichtungen auch eine gute UV-Beständigkeit, sowie eine gute Haftung auf Metallen, mineralischen Untergründen und Glas sowie auf verschiedensten organischen Polymermaterialien. Diese Effekte lassen sich auch bei Zusatz von geringen Mengen der Metall-organischen Verbindungen zu organischen Coating-Materialien (Harze oder allgemein Monomere) in vorteilhafter Weise nutzen.

Ein weiteres Anwendungsgebiet sind die so genannten "Improved Polymer Materials". Hier wird durch Zusatz der beschriebenen Verbindungen eine bessere und/oder schnellere Aushärtung von Kunststoffen oder Harzen erreicht. Hierbei können diese dazu dienen, die Viskosität der Materialien auch während des Härteprozesses zu variieren.

Auch im Gemisch mit anderen Metallverbindungen, z.B. Metallalkoholaten oder Metallsalzen, wie zum Beispiel mit Ti, Zr oder Si, entstehen Beschichtungen mit den unter dargestellten hervorragenden Eigenschaften.

Durch die Polymerisation aus einer Lösung oder Emulsion lassen sich polymere Partikel herstellen. Dies gelingt z.B. durch UV-initiierte Suspensions-/Emulsions-/Lösungspolymerisation auch aus wässerigen Systemen.

Die beschriebenen Metall-organischen Verbindungen können z.B. in Sol-Gel-Reaktionen genutzt werden, z.B. gemäß nachstehende Reaktionsschema zur Umsetzung der beschriebenen Verbindungen am Beispiel von Al-acrylat:

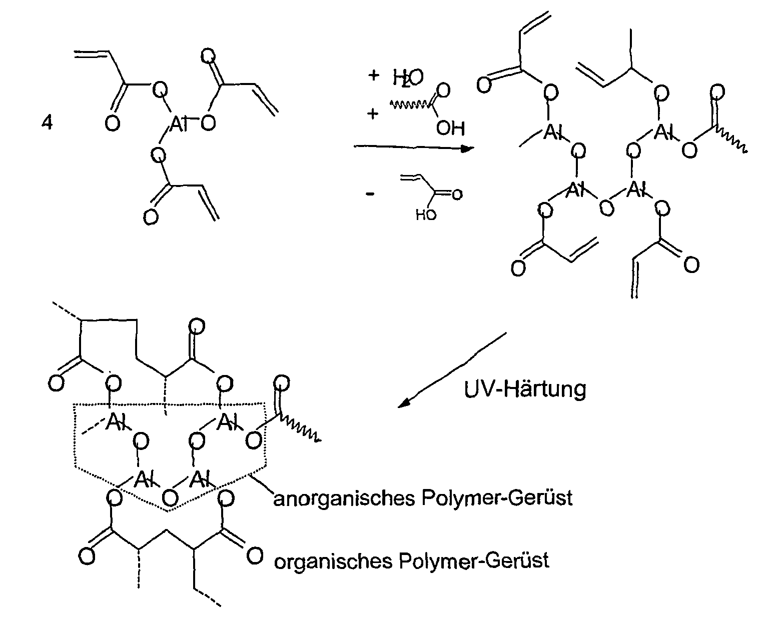

Folgende Anwendungsgebiete sind für die beschriebenen Metall-organischen Verbindungen in vorteilhafter Weise möglich:
- Monomer oder Co-Monomer in Polymerisationsprozessen, z.B. zur Herstellung, Beschichtung oder Modifikation von Formkörper und/oder Folien oder in der Herstellung von synthetischen Kautschuken,
- Additiv in Farben und Lacken,
- Trägermaterial für Pigmente und Farbstoffe,
- Komponente in UV-härtenden Klebern oder Kunststoffen,
- in der Herstellung von Kammpolymeren,
- zur Herstellung oder Modifikation von organischen und/oder anorganischen Materialien, wie z.B. Ionentauschern, Katalysatoren und Trägermaterialien, auch für chromatographische Anwendungen,
- Verkapselung von organischen Komponenten durch Beschichtung der Oberfläche,
- Herstellung elektrisch leitfähiger Materialien, z.B. für Anti-Statik-Ausrüstungen, in Membranen oder zur Herstellung von Platinen,
- als Additiv zur Verbesserung der Resistenz gegenüber Chemikalien und Umwelteinflüssen, als Haftverbesserer (z.B. für mineralische Putze) oder zur Verbesserung des Fließverhaltens (z.B. von Beton) und zur Versiegelung schützenswerter Objekte,
- Kratzfest- und/oder Hartbeschichtung, z.B. für Metalle, Keramiken, Hölzer oder Holz-basierten Produkten, Kunststoffen und Gläsern,
- zur Beschichtung oder Behandlung von Leder, Glas, Papier, Pappe, Kunststoffen, Metallen und Textilien,
- als Hydrophilierungs- oder Hydrophobierungsmittel in Form von Beschichtungen oder als Additiv,
- zur Herstellung von Antihaftbeschichtungen,
- als Grundstoff zur Herstellung von so genannten "anti-fouling"-Anstrichen für Schiffe oder maritime Bauwerke,
- zum Einsatz als Schutzanstrich in der Forstindustrie z.B. als "Wundpflaster" von Bäumen und/oder in der Agrarindustrie zum Schutz vor Schädlingen,
- als strahlenhärtendes Monomer in Druckfarben,
- als Rheologie- modifizierendes Additiv
- in Barriere-Coatings gegen den Durchtritt von Sauerstoff, Wasser oder zum Schutz vor mikrobiologischem Befall,
- zur Herstellung abrasiver Materialien,
- als Coating, Kleber oder Therapeutikum in der Pharmazie und/oder Medizintechnik, z.B. als Zahnzement, als Versiegelungslack für Zähne oder zur Retardisierung von Medikamenten,
- als Co-Komponente mit basischen Füllstoffen, wie z.B. Glas, können die beschriebenen Verbindungen zu Materialien mit besonderer Härte und Beständigkeit führen,
- als Entschäumer oder zur Herstellung von entschäumend wirkenden Produkten,
- zur Herstellung und/oder Modifikation von anorganischen Produkten, die funktionelle Gruppen enthalten können und
- zur Herstellung und Modifikation von Keramiken.

### Beispiele

### Herstellung von flüssigen Metallsalzen ungesättigter Carbonsäuren

Die Versuche wurden in einem 500 ml Reaktionskolben aus Glas, ausgestattet mit einem Thermometer, einem Destillationsaufsatz, Tropftrichter, Rührer und Gaszuleitung durchgeführt. Das erste Versuchsbeispiel wurde unter Stickstoffabdeckung, das zweite unter Pressluftdurchleitung durchgeführt. Zur Abführung der Reaktionswärme wurde das Reaktionsgefäß mit Hilfe eines Wasserbades gekühlt. Der Sauerstoffgehalt des zugeführten Gases in den Beispielen 2 bis 7 betrug zwischen 15 und 30 Vol% (ca. 21 Vol.%). Für die Beispiele 8 bis 10 lag die Reaktionsmischung sauerstoffgesättigt vor.

### Beispiel 1: Herstellung eines Aluminium-triacrylates in Lösung (Vergleichsbeispiel)

In den Kolben wurden 123,1 g Aluminium-tri-sek.-butanolat, 119,9 g Diethylenglycolmonobutylether, 0,7 g 4-Methoxyphenol und 2 g Kupferspäne vorgelegt, dazu wurden 108 g Acrylsäure bei Raumtemperatur (25 °C) innerhalb 1 Stunde und 4 Minuten mittels Tropftrichter zugetropft. Dabei stieg die Temperatur durch die Reaktionswärme auf 36 °C an. Mit Hilfe des Wasserbades wurde das Gemisch auf 25°C heruntergekühlt und 23 Minuten rühren gelassen bis die Temperatur wieder anstieg und das Produkt gelierte. Das Produkt polymerisierte unerwünschterweise, obwohl bei der Reaktion polymerisationshemmende Stoffe wie Kupferspäne und 4-Methoxyphenol zugegeben wurden.

### Beispiel 2: Herstellung eines Aluminium-triacrylates in Lösung

In den Kolben wurden 102,1 g Aluminium-tri-isopropanolat, 120,1 g Diethylenglycolmonobutylether, 0,7 g 4-Methoxyphenol vorgelegt, dazu wurden 108 g Acrylsäure bei Raumtemperatur (25 °C) innerhalb 15 Minuten mittels Tropftrichter zugetropft. Dabei stieg die Temperatur durch die Reaktionswärme auf 53 °C an. Mit Hilfe des Wasserbades wurde das Gemisch auf 39 °C heruntergekühlt. Das Produkt blieb flüssig und stabil, daher konnte es wie folgt weiterbehandelt werden. Es wurde innerhalb von 30 Minuten auf 60 °C aufgeheizt, und mittels eines kontinuierlich eingestellten Vakuums bis auf einen Wert von 243 mbar innerhalb von 2 Stunden das Co- Produkt Isopropanol ausgetrieben.

### Beispiel 3: Herstellung eines Aluminium-mono-acrylat-di-isopropanolates in Lösung

In den Kolben wurden 142,9 g Aluminium-tri-isopropanolat, 151,3 g Diethylenglycolmonobutylether, 0,7 g 4-Methoxyphenol vorgelegt, dazu wurden 50,4 g Acrylsäure bei Raumtemperatur (25 °C) innerhalb 8 min mittels Tropftrichter zugetropft. Dabei stieg die Temperatur durch die Reaktionswärme auf 55 °C an. Das Produkt blieb flüssig und stabil und konnte daher innerhalb von 30 Minuten auf 60 °C aufgeheizt werden. Mittels eines kontinuierlich eingestellten Vakuums bis auf einen Wert von 245 mbar wurde dann innerhalb von 38 Minuten das Co- Produkt Isopropanol ausgetrieben.

### Beispiel 4: Herstellung eines Al-mono-acrylat-di-ethylacetoacetates in Lösung

In den Kolben wurden 81,7 g Aluminium-tri-isopropanolat, 0,7 g 4-Methoxyphenol vorgelegt, dazu wurden 104,1 g Ethylacetoacetat bei einer Temperatur von 50 °C innerhalb 8 Minuten mittels Tropftrichter zugetropft. Dabei stieg die Temperatur durch die Reaktionswärme auf 61 °C an. Mit Hilfe des Wasserbades wurde das Gemisch auf 32°C abgekühlt. Dann wurde 142,6 g Diethylenglycolmonobutylether im Schuss dazugegeben. Anschließend wurde 28,8 g Acrylsäure schnell mittels Tropftrichter zugetropft. Die Temperatur stieg dabei um 4 °C an. Das Produkt blieb flüssig und stabil und konnte daher innerhalb von 30 Minuten auf 60°C aufgeheizt werden. Mittels eines Vakuums bis auf einen Wert von 200 mbar wurde innerhalb 1 Stunde das Co- Produkt Isopropanol ausgetrieben.

### Beispiel 5: Herstellung eines Zirkonium-tetra-acrylates in Lösung

In den Kolben wurden 509,3 g Zirkonium-tetra-butanolat, 498,6 g Diethylenglycolmonobutylether und 2,8 g 4-Methoxyphenol vorgelegt. Dann wurden 382,7 g Acrylsäure schnell mittels Tropftrichter zugetropft. Die Temperatur stieg dabei auf 45 °C an. Das Produkt blieb flüssig und stabil und konnte daher innerhalb von 30 Minuten auf 90 °C aufgeheizt werden. Mittels eines Vakuums bis auf einen Wert von 200 mbar wurde innerhalb 1 Stunde das Co-Produkt Butanol ausgetrieben.

### Beispiel 6: Herstellung eines Titan-tetra-acrylates in Lösung (nicht erfindungsgemäß)

In den Kolben wurden 510,6 g Titan-tetra-butanolat, 498,6 g Diethylenglycolmonobutylether und 2,8 g 4-Methoxyphenol vorgelegt. Dann wurden 432,6 g Acrylsäure schnell mittels Tropftrichter zugetropft. Die Temperatur stieg dabei auf 50 °C an. Das Produkt blieb flüssig und stabil und konnte daher innerhalb von 30 Minuten auf 90 °C aufgeheizt werden. Mittels eines Vakuums bis auf einen Wert von 200 mbar wurde innerhalb 1 Stunde das Co-Produkt Butanol ausgetrieben.

### Beispiel 7: Herstellung eines Magnesium-di-acrylates in Lösung (nicht erfindungsgemäß)

In den Kolben wurden 510,55 g Magnesium-di-butanolat, 498,6 g Diethylenglycolmonobutylether und 2,8 g 4-Methoxyphenol vorgelegt. Dann wurden 431,7 g Acrylsäure schnell mittels Tropftrichter zugetropft. Die Temperatur stieg dabei auf etwa 48 °C an. Das Produkt blieb flüssig und stabil und konnte daher innerhalb von 30 Minuten auf 90 °C aufgeheizt werden. Mittels eines Vakuums bis auf einen Wert von 200 mbar wurde innerhalb 1 Stunde das Co-Produkt Butanol ausgetrieben.

### Herstellung von festen Metallsalzen ungesättigter Carbonsäuren

Zusätzlich wurden Versuche durchgeführt, um die Metallverbindungen der ungesättigten Carbonsäuren als Feststoff herzustellen.
Allgemeine Vorgehensweise / Apparatur:
Die Versuche wurden in einem 1000 ml Reaktionskolben aus Glas am Rotationsverdampfer und angeschlossener Vakuumpumpe durchgeführt. Mit Hilfe des angelegten Vakuums von 750 mbar wurde die Acrylsäure durch ein Glasrohr in den Kolben gezogen.

### Beispiel 8: Herstellung eines Aluminiumtriacrylates, Iösemittelfrei

In den Kolben wurden 204,2 g Aluminium-tri-isopropanolat vorgelegt, dazu wurden 216 g Acrylsäure bei Raumtemperatur (25 °C) innerhalb 8 Minuten auf oben beschriebene Weise zugegeben. Dabei stieg die Temperatur durch die Reaktionswärme auf 32 °C an, und es entstand ein weißer Feststoff. Danach wurde die Heizung des Rotationsverdampfers eingeschaltet und schrittweise auf 70 °C erhitzt. Gleichzeitig wurde der Unterdruck schrittweise auf 24 mbar eingestellt. Auf diese Weise wurde das Isopropanol ausgetrieben und ein weißer Feststoff in Pulverform erhalten.

### Beispiel 9: Herstellung eines Aluminium-monoacrylat-diisopropanolates, Iösemittelfrei

In den Kolben wurden 204,2 g Aluminium-tri-isopropanolat vorgelegt, dazu wurden 72 g Acrylsäure bei Raumtemperatur (25 °C) innerhalb 8 Minuten auf oben beschriebene Weise zugegeben. Dabei stieg die Temperatur durch die Reaktionswärme auf 32 °C an, und es entstand ein weißer Feststoff. Danach wurde die Heizung des Rotationsverdampfers eingeschaltet und schrittweise auf 70°C erhitzt. Gleichzeitig wurde der Unterdruck schrittweise auf 24 mbar eingestellt. Auf diese Weise wurde das Isopropanol ausgetrieben und man erhielt den weißen Feststoff in Pulverform.

Zur besseren Übertragung in den großtechnischen Bereich wurden Versuche zur Herstellung des Produktes in Lösung unter gleichen Bedingungen in einem 5 1 Edelstahlreaktor durchgeführt. Bedingt durch den Einsatz von Pumpen zur Zuführung der Säuren wurde die Einspeisezeit gegenüber den Versuchen mit Glasgefäßen auf 2 Stunden erhöht.

### Beispiel 10: Herstellung eines Zirkonium-tetraacrylat, Iösemittelfrei

In den Kolben wurden 1021,3 g Zirkonium-tetrabutylat vorgelegt, dazu wurden 767,5 g Acrylsäure bei Raumtemperatur (25 °C) innerhalb 8 Minuten auf oben beschriebene Weise zugegeben. Dabei stieg die Temperatur durch die Reaktionswärme auf 35 °C an, und es entstand ein weißer Feststoff. Danach wurde die Heizung des Rotationsverdampfers eingeschaltet und schrittweise auf 90 °C erhitzt. Gleichzeitig wurde der Unterdruck schrittweise auf 20 mbar eingestellt. Auf diese Weise wurde das Butanol ausgetrieben und man erhielt den weißen Feststoff in Pulverform.

Die in den Beispielen 2 bis 10 hergestellten Metallsalze der Acrylsäure sind farblos und nahezu geruchlos im Gegensatz zur stechend riechenden Acrylsäure.

### Formulierung 1

Formulierung 1 besteht aus 50 % Aluminiumtriacrylat und 50 % Butyltriglykol. Es entstand eine klare, leicht gelbliche, viskose Flüssigkeit.

### Formulierung 2

Formulierung 2 besteht aus 50 % Aluminiumtrimethacrylat und 50 % Butyltriglykol. Es entstand eine klare, leicht gelbliche, viskose Flüssigkeit.

### Formulierung 3

Formulierung 3 besteht aus 31 % Aluminiumtriacrylat, 45 % n-Butylacrylat und 24 % 2-Propanol. Es entstand eine klare, leicht viskose Flüssigkeit.

### Formulierung 4

Formulierung 4 besteht aus 30 % Aluminiumtriacrylat, 32 % 2-Hydroxyethylmethacrylat und 38 % 2-Butanol. Es entstand eine klare, leicht viskose Flüssigkeit.

### Formulierung 5

Formulierung 5 besteht aus 35 % Aluminiumtrimethacrylat, 43 % n-Butylacrylat und 22 % 2-Propanol. Es entstand eine klare, leicht viskose Flüssigkeit.

### Formulierung 6

Formulierung 6 besteht aus 21 % Aluminiumtriacrylat, 31 % n-Butylacrylat und 48 % Hydroxyethylmethacrylat. Es entsteht eine klare, leicht viskose Flüssigkeit.

### Formulierung 7 (nicht erfindungsgemäß)

Formulierung 7 besteht aus 90 % Formulierung 3 und 10 % Tetra-n-butylzirconat. Es entstand eine klare, leicht viskose Flüssigkeit.

### Formulierung 8 (nicht erfindungsgemäß)

Formulierung 8 besteht aus 70 % Formulierung 3 und 30 % Tetra-n-butylzirconat. Es entstand eine klare, leicht viskose Flüssigkeit.

### Formulierung 9 (nicht erfindungsgemäß)

Formulierung 9 besteht aus 90 % Formulierung 3 und 10 % Tetra-n-butyltitanat. Es entstand eine klare, leicht gelbliche, leicht viskose Flüssigkeit.

### Formulierung 10 (nicht erfindungsgemäß)

Formulierung 10 besteht aus 70 % Formulierung 3, 30 % Tetra-n-butyltitanat. Es entstand eine klare, leicht gelbliche, leicht viskose Flüssigkeit.

Alle Formulierungen 1 bis 10 beinhalten noch 3 Gew.% Photoinitiator (2,2-Diethoxyacetophenon) und ca. 0,15-0,2 Gew.% Stabilisator (4-Methoxy-phenol).

In den folgenden Anwendungsbeispielen (Anwendung 11 - 18) wurden als Referenzsubstanzen zu den getesteten, oben aufgeführten Formulierungen ein handelsüblicher Acryl-Buntlack (weiß, glänzend, Acryl-Buntlack der Baumarktkette Hornbach), sowie ein UV-härtendes Klebergel (UV-1-Phasen-Gel der Firma Wilke Cosmetics) verwendet.

Beschichtungen mit dem Acryllack härteten durch Lufttrocknung und wurden keiner UV-Härtung unterzogen.

### Anwendung 11 - Ritzhärte nach Wolff-Wilborn (ISO 15184)

Die Ritzhärte nach Wolff-Wilborn (ISO 15184) wurde mit Hilfe von Bleistiften unterschiedlicher Härte bestimmt. Die Bleistifte wurden mit einem Schlitten, unter dem gleichen Winkel, der eine gleichbleibende Kraft auf unterschiedliche Probeträger ausübt, über die Probefläche gezogen. Die Filmhärte wurde bestimmt durch die beiden Härtegrade an der Grenze zwischen Schreib- und Eindringeffekt. Die Bleistifthärten variierten von 6B bis 9H.

**Tabelle 1**

| | Glas | Polycarbonat | Polypropylen | Stahl | Aluminium |
|---|---|---|---|---|---|
| unbeschichtet | >9H | 2B/B | B/HB | 8H/9H | 3B/2B |
| UV-Klebergel | HB/F | <6B | <6B | 2B/B | 3B/2B |
| Acryllack (Lufttrocknung) | F/H | F/H | 3B/2B | F/H | H/2H |
| Formulierung 3 | H/2H | HB/F | 2B/B | 7H/8H | 6H/7H |
| Formulierung 6 | F/H | HB/F | 4B/3B | --- | H/2H |
| Formulierung 7 | F/H | --- | --- | X | 2B/B |
| Formulierung 8 | --- | --- | --- | X | --- |
| Formulierung 9 | --- | --- | --- | X | H/2H |
| Formulierung 10 | --- | --- | --- | X | --- |

| | | | | | |
|---|---|---|---|---|---|
| *Mit X gekennzeichnete Tabellenfelder bedeuten, dass der Probekörper nicht mit der dazugehörigen Beschichtung getestet wurde; "---" bedeutet*, *dass sich der Film vom Probeträger löste und die Probe nicht mehr zu vermessen war. Härteskala der verwendeten Bleistifte von weich nach hart: 6B, 5B, 4B, 3B, 2B, B, HB, F, H, 2H, 3H, 4H, 5H, 6H, 7H, 8H, 9H.* | | | | | |

Nach 7-tägiger Lagerung nach der UV-Härtung erzielte Formulierung 3 den besten Wert auf Stahl, Glas, Polypropylen und Aluminium. Die Ritzhärte der Beschichtungen von Formulierung 3 auf Polypropylen und Aluminium übertrafen den Wert für unbeschichtetes Material. Für das Polycarbonat ergab die Beschichtung mit dem Acryllack das beste Ergebnis.

### Anwendung 12 - Die Ritzhärte nach Clemen (ISO 1518)

Die Ritzhärte nach Clemen (ISO 1518) beschreibt die Ritzbeanspruchung durch Bewegung der Probe unter einem mit definierter Kraft (0-20 N) belasteten kugelförmigen Hartmetallstichel. Die Ritzhärte entspricht der zum vollständigen Durchritzen der Beschichtung erforderlichen Kraft. Für die Ritzhärte nach Clemen ergaben sich nach einer 7-tägigen Lagerzeit nach UV-Härtung folgende Ergebnisse:

**Tabelle 2**

| | Glas | Polycarbonat | Polypropylen | Stahl | Aluminium |
|---|---|---|---|---|---|
| UV-Klebergel | 10 | 5 | --- | 15 | 13 |
| Acryllack (Lufttrocknung) | 3 | 4 | 1 | 16 | 12 |
| Formulierung 3 | 10 | 5 | 2 | 12 | 4 |
| Formulierung 6 | 4 | 6 | 1 | --- | 5 |
| Formulierung 7 | 4 | --- | --- | X | 2 |
| Formulierung 8 | --- | --- | --- | X | --- |
| Formulierung 9 | --- | --- | --- | X | 17 |
| Formulierung 10 | --- | --- | --- | X | --- |

| | | | | | |
|---|---|---|---|---|---|
| *Mit X gekennzeichnete Tabellenfelder bedeutet, dass der Probekörper nicht mit der dazugehörigen Beschichtung getestet wurde; "---" bedeutet, dass sich der Film vom Probeträger löste und die Probe nicht mehr zu vermessen war.* | | | | | |

Der als Referenzsubstanz dienende Acryllack hatte die beste Ritzhärte auf Stahl und erreichte auch einen guten Vergleichswert auf Aluminium. Die beste Ritzhärte auf Aluminium zeigte Formulierung 9. Der Acryllack, Formulierung 3, Formulierung 6 und das UV-härtende Klebergel ergaben auf Polycarbonatkörpern eine geringe Ritzhärte. Formulierung 3 und das UV-Klebergel erreichten auf Glas die beste Ritzhärte. Auf dem Polypropylenprobekörper erreichten Acryllack, Formulierung 3 und Formulierung 6 nur eine geringe Ritzhärte.

### Anwendung 13 - Pendelhärte nach König (DIN 53157, ISO 1522)

Die Dämpfung eines auf der Beschichtung schwingenden Pendels wird gemäß Normvorschrift bewertet. Das Pendel ruhte auf zwei Stahlhalbkugeln, die auf der zu prüfenden Beschichtung in Schwingung gebracht werden. Eine hohe Schwingungszahl ist gleichbedeutend mit einer hohen Härte.

**Tabelle 3**

| | Glas | Polycarbonat | Polypropylen | Stahl | Aluminium |
|---|---|---|---|---|---|
| UV-Klebergel | 48 | 56 | 25 | 42 | 32 |
| Acryllack (Lufttrocknung) | 141 | 196 | 113 | 83 | 132 |
| Formulierung 3 | 180 | 202 | --- | 162 | 206 |
| Formulierung 6 | 160 | 191 | 115 | --- | 189 |
| Formulierung 7 | 119 | --- | --- | X | 179 |
| Formulierung 8 | --- | --- | --- | X | --- |
| Formulierung 9 | --- | --- | --- | X | 193 |
| Formulierung 10 | --- | --- | --- | X | 182 |

| | | | | | |
|---|---|---|---|---|---|
| *Mit X gekennzeichnete Tabellenfelder bedeuten, dass der Probekörper nicht mit der dazugehörigen Beschichtung getestet wurde, "---" bedeutet, dass sich der Film vom Probeträger löste und die Probe nicht mehr zu vermessen war.* | | | | | |

Formulierung 3 zeigte auf Glas, Polycarbonat, Stahl und Aluminium die höchsten Pendelhärten. Auf Polypropylen erreichten Formulierung 6 und der Acryllack gute Ergebnisse, während Formulierung 3 sich vom Probeträger löste.

### Anwendung 14 - Zeitlicher Verlauf

Die Vorteile der beschriebenen Formulierungen werden deutlicher, wenn unterschiedliche Zeiträume in die Auswertung einfließen.

Es zeigte sich, dass die eingesetzten Formulierungen sowohl sehr schnell (Formulierung 3), als auch nach Lagerung (Formulierung 6) gute Ergebnisse lieferten. Dies ist für den Einsatz in unterschiedlichen Anwendungen von Vorteil.

**Tabelle 4**

| Ritzhärte nach Clemen (ISO 1518) | Glas | | | Polycarbonat | | | Polypropylen | | | Stahl | | | Aluminium | | |
|---|---|---|---|---|---|---|---|---|---|---|---|---|---|---|---|
| | Lagerzeit nach UV-Härtung | | | | | | | | | | | | | | |
| | 1h | 1T | 7T | 1h | 1T | 7T | 1h | 1T | 7T | 1h | 1T | 7T | 1h | 1T | 7T |
| UV-Klebergel | 12 | 9 | 10 | 5 | 11 | 5 | 3 | 3 | --- | 19 | 16 | 15 | 11 | 11 | 13 |
| Acryllack (Lufttrock.) | 5 | 4 | 3 | 4 | 4 | 4 | 1 | 1 | 1 | 10 | 10 | 16 | 6 | 7 | 12 |
| Formul. 1 | 4 | 6 | 12 | 5 | 4 | --- | <1 | --- | --- | 4 | 4 | --- | 5 | 4 | --- |
| Formul. 3 | 4 | 8 | 10 | 2 | 7 | 5 | 2 | 2 | 2 | 3 | 7 | 10 | 5 | 6 | 4 |
| Formul. 6 | 1 | 6 | 4 | 2 | 5 | 6 | 1 | 1 | 1 | 1 | 4 | --- | 1 | 5 | 5 |
| Formul. 7 | 2 | 2 | 5 | 3 | 2 | --- | 3 | 2 | --- | X | X | X | 2 | 2 | 2 |
| Formul. 8 | --- | --- | --- | --- | --- | --- | --- | --- | --- | X | X | X | --- | --- | --- |
| Formul. 9 | 5 | 2 | --- | --- | --- | --- | --- | --- | --- | X | X | X | 10 | 13 | 17 |
| Formul. 10 | 5 | 7 | --- | --- | --- | --- | --- | --- | --- | X | X | X | 2 | 4 | --- |

**Tabelle 5**

| Pendelhärte nach König (DIN 53157; ISO 1522) | Glas | | | Polycarbonat | | | Polypropylen | | | Stahl | | | Aluminium | | |
|---|---|---|---|---|---|---|---|---|---|---|---|---|---|---|---|
| | Lagerzeit nach UV-Härtung | | | | | | | | | | | | | | |
| | 1h | 1T | 7T | 1h | 1T | 7T | 1h | 1T | 7T | 1h | 1T | 7T | 1h | 1T | 7T |
| UV-Klebergel | 55 | 48 | 48 | 42 | 91 | 56 | 25 | 45 | 25 | 22 | 56 | 42 | 20 | 50 | 32 |
| Acryllack (Lufttrock.) | 83 | 127 | 141 | 167 | 183 | 196 | 85 | 118 | 113 | 34 | 69 | 83 | 74 | 134 | 132 |
| Formul. 1 | 137 | 174 | --- | 200 | 199 | 182 | 105 | --- | --- | 129 | 158 | 123 | 118 | 181 | 133 |
| Formul. 3 | 147 | 179 | 180 | 207 | 220 | 202 | 127 | 111 | --- | 136 | 160 | 162 | 185 | 188 | 206 |
| Formul. 6 | 24 | 118 | 160 | 35 | 144 | 164 | 49 | 90 | 115 | 27 | 111 | --- | 27 | 120 | 189 |
| Formul.7 | 105 | 136 | 119 | 69 | 81 | --- | 89 | 92 | --- | X | X | X | 115 | 153 | 179 |
| Formul. 8 | --- | --- | --- | --- | --- | --- | --- | --- | --- | X | X | X | --- | --- | --- |
| Formul. 9 | 121 | 179 | --- | --- | --- | --- | --- | --- | --- | X | X | X | 176 | 176 | 193 |
| Formul. 10 | 183 | 189 | --- | --- | --- | --- | --- | --- | --- | X | X | X | 186 | 190 | 182 |

**Tabelle 6**

| Ritzhärte nach Wolff-Wilborn (ISO 15184) | Glas | | | Polycarbonat | | | Polypropylen | | | Stahl | | | Aluminium | | |
|---|---|---|---|---|---|---|---|---|---|---|---|---|---|---|---|
| | Lagerzeit nach UV-Härtung | | | | | | | | | | | | | | |
| | 1h | 1T | 7T | 1h | 1T | 7T | 1h | 1T | 7T | 1h | 1T | 7T | 1h | 1T | 7T |
| UV-Klebergel | 3B/ 2B | B/ HB | HB /F | 4B/ 3B | 3B/ 2B | > 6B | 6B/ 5B | > 6B | > 6B | 3B/ 2B | HB /F | 2B /B | 2B /B | 3B/ 2B | 3B/ 2B |
| Acryllack (Lufttrock.) | B/ HB | HB /F | F / H | HB /F | F/ H | F/ H | 3B/ 2B | 3B/ 2B | 3B/ 2B | 3B/ 2B | HB /F | F/ H | B/ HB | HB /F | H/ 2H |
| Formul. 3 | B/ HB | HB /F | H/ 2H | F/ H | B/ HB | HB /F | 2B/ B | 2B/ B | 2B/ B | F/ H | H/ 2H | 7H/ 8H | F/ H | H/ 2H | 6H/ 7H |
| Formul.6 | < 6B | B/ HB | F/ H | 6B/ 5B | B/ HB | HB /F | < 6B | 5B/ 4B | 4B/ 3B | 6B/ 5B | B/ HB | --- | 6B/ 5B | H/ 2H | H/ 2H |
| Formul. 7 | F/ H | F/ H | F/ H | 5B/ 4B | 5B/ 4B | --- | 4B/ 3B | 4B/ 3B | --- | X | X | X | 2H/ 3H | 2H/ 3H | 2B/ B |
| Formul. 8 | --- | --- | --- | --- | --- | --- | --- | --- | --- | X | X | X | --- | --- | --- |
| Formul.9 | F/ H | F/ H | --- | --- | --- | --- | --- | --- | --- | X | X | X | 6B/ 5B | 6B/ 5B | H/ 2H |
| Formul. 10 | --- | --- | --- | --- | --- | --- | --- | --- | --- | X | X | X | HB /F | F/ H | H/ 2H |

| | | | | | | | | | | | | | | | |
|---|---|---|---|---|---|---|---|---|---|---|---|---|---|---|---|
| *Mit X gekennzeichnete Tabellenfelder bedeuten, dass der Probekörper nicht mit der dazugehörigen Beschichtung getestet wurde; "---" bedeutet, dass sich der Film vom Probeträger löste und die Probe nicht mehr zu vermessen war.* | | | | | | | | | | | | | | | |

### Anwendung 15 - Tesa^{®}-Abriss (Haftung)

Es wurde ein Streifen Tesa^{®-}Film auf die Beschichtung aufgebracht und anschließend wieder ruckartig entfernt. Nun wurde die Beschichtung begutachtet und in fünf Klassen eingeteilt - von 0 bis 4. Die Bedeutung der Klassen wird in der nachfolgenden Tabelle erklärt:

**Tabelle 7**

| Bewertung | 0 | 1 | 2 | 3 | 4 |
|---|---|---|---|---|---|
| Abriss in % | 0 (kein Abriss) | <25 | 50 | >50 | 100 (vollständiger Abriss der vom Klebestreifen bedeckten Fläche) |

Der Acryllack, Formulierung 3 und Formulierung 6 hafteten zu 100% auf folgenden Probekörpern: Glas, Stahl und Aluminium. Auf der Polycarbonatoberfläche haftete nur der UV-Klebergelfilm. Auf dem Polypropylenprobekörper wurden alle Beschichtungen zu 100% abgerissen.

**Tabelle 8**

| | Glas | Polycarbonat | Polypropylen | Stahl | Aluminium |
|---|---|---|---|---|---|
| UV-Klebergel | 0 | 0 | 4 | 0 | 4 |
| Acryllack (Lufttrocknung) | 0 | 4 | 4 | 0 | 0 |
| Formulierung 3 | 0 | 4 | 4 | 0 | 0 |
| Formulierung 6 | 0 | 4 | --- | 0 | 0 |
| Formulierung 7 | 0 | --- | --- | X | 2 |
| Formulierung 8 | --- | --- | --- | X | --- |
| Formulierung 9 | --- | --- | --- | X | 0 |
| Formulierung 10 | --- | --- | --- | X | 0 |

| | | | | | |
|---|---|---|---|---|---|
| *Mit X gekennzeichnete Tabellenfelder bedeuten, dass der Probekörper nicht mit der dazugehörigen Beschichtung getestet wurde; "---" bedeutet, dass sich der Film vom Probeträger löste und die Probe nicht mehr zu vermessen war.* | | | | | |

### Anwendung 16 - Gitterschnitt

Bei diesem Test wird die Elastizität von Beschichtungen gemessen. An genormten Stahllamellen wird mit einem scharfen Cutter entlang geschnitten. Es werden jeweils zwei Schnitte durchgeführt, die rechtwinkelig zueinander angeordnet sind, so dass sich ein Schachbrettmuster ergibt. Der Gitterschnitt hat eine ähnliche Klasseneinteilung wie der Tesa-Abriss, die Prozentverteilung ist anders gestaffelt.

**Tabelle 9**

| Bewertung | 0 | 1 | 2 | 3 | 4 |
|---|---|---|---|---|---|
| Abgeplatzte Schnittränder in % | Glatte Ränder | 5 | 15 | 35 | 65 |

Der Gitterschnitt wurde an Filmen getestet, die nach der UV-Härtung über einen Zeitraum von sieben Tagen in einem Klimaraum gelagert wurden. Das UV-Klebergel und der Acryllack zeigten auf Glas die besten Ergebnisse. Auf Polycarbonat erzielten der Acryllack und das UV-Klebergel sowie die Formulierungen 3 und 6 die besten Resultate. Auf Polypropylen erreichte nur der Film des Acryllackes ein gutes Ergebnis im Gitterschnitt. Formulierung 3 sowie das UV-Klebergel erreichten auf Stahl sehr gute Ergebnisse, während der Acryllack deutlich schlechter abschnitt. Auf Aluminium zeigten alle getesteten Formulierungen und Referenzsubstanzen sehr gute Film-Eigenschaften.

**Tabelle 10**

| | Glas | Polycarbonat | Polypropylen | Stahl | Aluminium |
|---|---|---|---|---|---|
| UV-Klebergel | 0 | 0 | 4 | 0 | 0 |
| Acryllack (Lufttrocknung) | 0 | 0 | 0 | 4 | 0 |
| Formulierung 3 | 1 | 0 | --- | 0 | 0 |
| Formulierung 6 | 1 | 0 | --- | --- | 0 |
| Formulierung 7 | 2 | --- | --- | X | 0 |
| Formulierung 9 | --- | --- | --- | X | 0 |
| Formulierung 10 | --- | --- | --- | X | 0 |

| | | | | | |
|---|---|---|---|---|---|
| *Mit X gekennzeichnete Tabellenfelder bedeuten, dass der Probekörper nicht mit der dazugehörigen Beschichtung getestet wurde; "---" bedeutet, dass sich der Film vom Probeträger löste und die Probe nicht mehr zu vermessen war.* | | | | | |

### Anwendung 17 - Scheuerfestigkeit

Die Scheuerfestigkeit wurde mit einem handelsüblichen Scheuerschwamm getestet. Dazu wurde der Schwamm in einen Schlitten gespannt, der maschinell vor und zurück bewegt wurde. Ein Zyklus entspricht hierbei einer Vor- und Rückbewegung des Schlittens. Als Beurteilungskriterium diente die Anzahl der Zyklen, die der Schwamm über den Film gezogen wurde, ohne ihn zu zerstören. Der Test wurde nach max. 5000 Zyklen gestoppt. Das Gewicht des Schwamms und des Schlittens betrug 134 g, dies entspricht einem Druck von 4 g/cm². Die Filmdicke der getesteten Formulierungen und Referenzsubstanzen betrug 50 µm nass . Die Lagerung nach der UV-Härtung erfolgte bei Normbedingungen.

Formulierung 3 erreichte, ebenso wie das UV-Klebergel, für alle getesteten Lagerzeiten eine Scheuerfestigkeit von >5000 Zyklen. Die Filme wiesen Scheuerspuren auf, waren aber noch intakt. Formulierung 6 verbesserte mit zunehmender Lagerzeit die Scheuerfestigkeit. Der Acryllack verlor mit zunehmender Lagerzeit an Scheuerfestigkeit.

**Tabelle 11**

| | Zyklen nach 1 Stunde Lagerzeit | Zyklen nach 24 Stunden Lagerzeit | Zyklen nach sieben Tagen Lagerzeit |
|---|---|---|---|
| Acryllack (Lufttrocknung) | 2400 | 3200 | 1500 |
| UV-Klebergel | >5000 | >5000 | >5000 |
| Formulierung 3 | >5000 | >5000 | >5000 |
| Formulierung 6 | 840 | 1200 | 4500 |

### Anwendung 18 - Enthaftungsprüfung (angelehnt an ASTM D 4541)

**Tabelle 12**

| Probekörper | UV-Klebergel | | Formulierung 3 | | Acryllack |
|---|---|---|---|---|---|
| | Lagerzeit nach UV-Härtung | | | | Lufttrocknung |
| | 1 Tag | 7 Tage | 1 Tag | 7 Tage | 14 Tage |
| Glas | 2,0 | >3,5 | 3,5 | 2,5 | 1,0 |
| Polypropylen | <0,5 | <0,5 | <0,5 | <0,5 | X |
| Polycarbonat | 0,5 | <0,5 | 0,5 | 1,0 | X |
| PVC | <0,5 | 0,5 | <0,5 | 0,5 | X |
| Stahl | 1,0 | 1,0 | 1,25 | 1,25 | X |
| Aluminium | 0,5 | 0,5 | 0,5 | 0,5 | 1,0 |
| Kupfer | 0,5 | 0,5 | 1,0 | 1,0 | X |

| | | | | | |
|---|---|---|---|---|---|
| *Mit X gekennzeichnete Tabellenfelder bedeuten, dass der Probekörper nicht mit der dazugehörigen Beschichtung getestet wurde.* | | | | | |

Die Haftfestigkeitsprüfung durch Abreißversuche wurde nach folgender Methode vorgenommen. Auf den im Klimaraum gelagerten Film werden sorgfältig entfettete bzw. geschmirgelte Prüfkörper, so genannte "Dolly's" mit einem 2-Komponenten-Epoxidkleber aufgeklebt. Überschüssiger Kleber wird entfernt. Die Aushärtezeit beträgt bei 23 °C 8 Stunden. Die am aufgeklebten Prüfstempel angreifende Zugspannung wurde kontinuierlich gesteigert, bis der Prüfkörper abriss (zahlenmäßige Erfassung der Haftfestigkeit). Nach ASTM D 4541 sind für ein aussagekräftiges Abreißexperiment mindestens drei Einzelversuche erforderlich. Das primäre Prüfergebnis ist die am Schleppzeiger in N/mm ablesbare Zugspannung, die zum Abriss geführt hat.

Überprüft wurden folgende Beschichtungen: Acryllack, UV-Klebergel und Formulierung 3.

Die Beschichtungen mit Formulierung 3 erzielte auf Stahl, Kupfer und Polycarbonat die höchsten Enthaftungswerte. Die Werte wurden bereits nach 1-tägiger Lagerung nach der UV-Härtung erreicht. Auf Polypropylen und PVC unterschieden sich die Beschichtungen mit dem UV-Klebergel und Formulierung 3 nicht voneinander. Auf Glas steigerte das UV-Klebergel nach 7-tägiger Lagerung seine Werte, die Werte von Formulierung 3 fielen dagegen ab.

### Anwendung 19 - Rheologie

Druckharze benötigen eine exakt eingestellte Rheologie, um einen Druckprozess mit hoher Geschwindigkeit realisieren zu können. Ein wichtiger Wert bei der Bestimmung rheologischer Daten ist die scheinbare Viskosität. Die Veränderung der scheinbaren Viskosität von Druckharzbindemitteln durch den Zusatz von unterschiedlichen Metallacrylat-haltigen Formulierungen wurde bestimmt. Als Referenzmaterial wurden ein unbehandeltes Druckfarbenbindemittel sowie ein mit DOROX D 515 versetztes Bindemittel eingesetzt.

Alle geprüften Proben ermöglichten die Einstellung der rheologischen Eigenschaften.

**Tabelle 13**

| Probe | Meß-temperatur [°C] | Al-Konzentration in Bindern [%] | scheinbare Viskosität bei 50s⁻¹ [Pa*s] | scheinbare Viskosität bei 5s⁻¹ [Pa*s] |
|---|---|---|---|---|
| Druckfarbenbindemittel (DFBM) | 20 | 0,000 | 134 | 152 |
| DFBM + DOROX-D515 | 20 | 0,060 | 204 | 292 |
| DFBM + DOROX-D515 | 20 | 0,080 | 226 | 362 |
| DFBM + DOROX-D515 | 20 | 0,100 | 269 | 491 |
| DFBM + Formul. 1 | 20 | 0,060 | 203 | 297 |
| DFBM + Formul. 1 | 20 | 0,080 | 284 | 495 |
| DFBM + Formul. 1 | 20 | 0,100 | 246 | 459 |
| DFBM + Formul. 2 | 20 | 0,060 | 205 | 295 |
| DFBM + Formul. 2 | 20 | 0,080 | 248 | 395 |
| DFBM + Formul. 2 | 20 | 0,100 | 278 | 473 |

### Anwendung 20 - Wasseraufnahme von Papier

Die Wasseraufnahme von Papier wurde mit handelsüblicher Papierqualität getestet. Hierzu wurden die Formulierungen 1, 3 und 6 in einer Schichtdicke von 50 µm (beidseitig auf dem Papier) getestet. Als Referenzmaterial diente unbehandeltes Papier. Die Papiere wurden eine Stunde nach der UV-Belichtung, für 2 Std. bzw. 24 Std. in ein Wasserbad gelegt. In einer weiteren Untersuchung wurden die Proben 7 Tage nach der UV-Belichtung erneut für zwei bzw. 24 Std. in ein Wasserbad gelegt. Die Wasseraufnahme wurde durch Differenzwägung ermittelt, die Angaben erfolgen in % Wasseraufnahme

Das Papier, das mit Formulierung 3 beschichtet war, erzielte bei der Untersuchung nach einer Stunde Lagerzeit nach UV-Belichtung die geringste Wasseraufnahme. Nach der 7-tägigen Lagerzeit erreichten die mit Formulierung 6 beschichteten Papiere die geringste Wasseraufnahme.

**Tabelle 14**

| | Wasseraufnahme in % durch Behandlung im Wasserbad | | | | | | | |
|---|---|---|---|---|---|---|---|---|
| | 2Std | 2Std | 24Std | 24Std | 2Std | 2Std | 24Std | 24Std |
| Unbe. Papier | 101,4 | 101,1 | 105,0 | 104,6 | --- | --- | --- | --- |
| | 1 Std. nach UV-Belichtung | | | | 7 Tage nach UV-Belichtung | | | |
| Formul. 1 | 38,4 | 38,3 | 41,7 | 49,1 | 65,6 | 74,6 | 72,3 | 82,7 |
| Formul. 3 | 37,2 | 37,0 | 37,6 | 36,6 | 56,0 | 56,0 | 57,8 | 57,9 |
| Formul. 6 | 40,7 | 39,7 | 44,2 | 43,5 | 33,6 | 30,2 | 41,5 | 39,4 |

### Anwendung 21 - UV-Belastung von Aluminiumacrylatformulierungen auf Glas

Bei diesem Test wurden die Formulierungen 3, 6 und 1 in einer Schichtdicke von 50 µm nass auf jeweils einen Glasprobeträger aufgebracht und durch UV-Strahlung gehärtet. Diese Glaskörper wurden bei ca. 54 °C über einen Zeitraum von 225 Stunden mit UV-Licht belastet. Die Auswertung erfolgte in Anlehnung an DIN 55980 in Form eines "Blau-Gelb"-Wertes. In dieser Nomenklatur bedeutet ein positiver Blau-Gelb-Wert einen Gelbstich, ein negativer einen Blaustich. Der Blindwert der genutzten Kalibrierungskachel betrug: +7,24.

**Tabelle 15**

| Probe | Start-Wert | Wert nach UV-Belastung |
|---|---|---|
| Formulierung 3 | +7,51 | +7,40 |
| Formulierung 6 | +7,44 | +7,54 |
| Formulierung 1 | +7,62 | +6,97 |

Der "Blau-Gelb-Wert" der UV-belasteten Filme war niedriger bzw. unwesentlich verändert gegenüber dem Wert, der zu Beginn der Messung erreicht wurde (Start-Wert). Dies bedeutet, dass die Beschichtungen nicht zur "Vergilbung" neigen.

### Anwendung 22 - Chemikalienbeständigkeit

Es wurden Formulierung 3, Formulierung 6, Formulierung 1 und das UV-Klebergel in einer Schichtdicke von 50 µm auf Glas aufgezogen und UV-gehärtet. Nach einer Lagerzeit von 1 Stunde und 24 Stunden wurden die Filme mit den folgenden Chemikalien behandelt: Methylethylketon, 2-Propanol, Essigsäure, Phosphorsäure, Mineralöl und 30%-ige Natronlauge. Die Chemikalien wirkten über einen Zeitraum von 2 Stunden auf die Filme ein.

**Tabelle 16**

| | MEK | | 2-Propanol | | Essigsäure | | Phosphorsäure | | Mineralöl | | Natronlauge 30% | |
|---|---|---|---|---|---|---|---|---|---|---|---|---|
| | Lagerzeit nach UV-Härtung | | | | | | | | | | | |
| | 1 h | 24 h | 1 h | 24 h | 1 h | 24 h | 1 h | 24 h | 1 h | 24 h | 1 h | 24 h |
| UV-Klebergel | - | + | - | + | - | - | - | - | + | + | +/- | +/- |
| Formul. 1 | +/- | a | + | a | - | - | - | - | +/- | a | - | - |
| Formul. 2 | - | - | +/- | - | - | - | - | - | +/- | + | - | - |
| Formul.3 | +/- | + | + | + | - | - | - | - | + | + | - | - |
| Formul. 6 | - | +/- | - | + | - | - | - | - | - | + | - | - |

| | | | | | | | | | | | | |
|---|---|---|---|---|---|---|---|---|---|---|---|---|
| *Zeichenerklärung: - = unbeständig;* +/- *= Film ist angegriffen aber ist noch intakt,* + = *beständig, α = abgeplatzt* | | | | | | | | | | | | |

Die Filme der Formulierung 3 waren bereits nach 1 Stunde Lagerzeit nach der UV-Härtung beständig bzw. teilweise beständig gegen Methylethylketon, 2-Propanol und Mineralöl. Das UV-Klebergel und die Formulierung 6 erreichten diese Beständigkeit erst nach einer Lagerzeit von 24 h (Ausnahme: der Film des UV-Klebergels war gegen Mineralöl ebenfalls nach 1 h Lagerzeit beständig). Das UV-Klebergel war gegenüber 30%-iger Natronlauge teilweise beständig. Keine der getesteten Substanzen war gegen Säuren beständig.

### Anwendung 23 - Beschichtung von Sportfunktionsfasern

Eine Beschichtung von Sportfunktionskleidung mit Formulierung 3 wurde zur Hydrophobierung der Faser vorgenommen. Auf der beschichteten und UV-belichteten Sportfunktionsfaser bildete das aufgesprühte Wasser Tropfen aus, die auf der Oberfläche des Stoffes verbleiben. Die unbehandelte Sportfunktionsfaser saugte das Wasser dagegen unmittelbar auf. Die Sportfunktionsfaser wurde mit einer Seifenlauge gewaschen, getrocknet und nach einer Lagerzeit von 3 Wochen erneut dem Sprühtest unterzogen. Das Ergebnis blieb unverändert positiv.

## Patentansprüche

1. Verfahren zur Herstellung von Metallsalzen kurzkettiger, ungesättigter Carbonsäuren durch Umsetzung
- von Metallalkoholat-Verbindungen
- mit Carbonsäuren der allgemeine Formel
CₙH₂ₙ₋₁C(=O)OH
mit Doppelbindung in 2- oder 3- Stellung, worin n für 2, 3, 4, 5 oder 6 steht, und/oder Maleinsäure,
- in Gegenwart von Sauerstoff (O₂), wobei man Sauerstoff stetig so zuführt dass die Reaktionslösung zumindest zu 50% sauerstoffgesättigt ist, und
die Metallsalze zumindest eine Gruppe der Formel
CₙH₂ₙ₋₁C(=O)O- und/oder -OC(=O)CH=CHC(=O)O-(H)
und folgende Metalle oder deren Mischungen
Al, Si, Sn, La, Zr, Cu und/oder Zn
aufweisen.

2. Verfahren gemäß Anspruch 1, **dadurch gekennzeichnet, dass** man Sauerstoff stetig so zuführt, dass die Reaktionslösung zumindest zu 90% sauerstoffgesättigt ist.

3. Verfahren gemäß Anspruch 1 oder 2, **dadurch gekennzeichnet, dass** die Metallsalze der allgemeinen Formel
M(OOCCₙH₂ₙ₋₁)ₐ(R¹)_{b}
entsprechen und durch Umsetzung einer linearen oder verzweigten, ungesättigten Carbonsäure der Formel
CₙH₂ₙ₋₁-COOH ,
wobei n gleich 2, 3, 4, 5 oder 6 ist mit Doppelbindung in 2- oder 3- Stellung mit einer Metallverbindung der allgemeinen Formel
M(R¹)_{c}
und ggf.
H(R¹)
worin
a zumindest 1; b 0, 1, 2 oder 3 ist und (a+b) und c unabhängig voneinander eine ganze Zahl von 2 bis 4 sind,
M für das Metall gemäß Anspruch 1 steht,
R¹ für eine Alkoholat-Gruppe mit einem C1- bis C6- Kohlenwasserstoffrest steht, wobei R¹ eine gesättigte, lineare oder verzweigte Alkoholat-Gruppe ist, herstellbar aus einem Alkohol mit zumindest einer -OH Gruppe,
oder 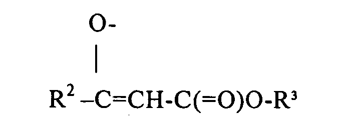 mit R² bzw. R³ gleich -CH₃, -C₂H₅, -C₃H₇ oder -C₄H₉, und
wobei n, R¹, R² und R³ für jedes a, b bzw. c unterschiedlich sein können und zumindest ein R¹ in M(R¹)_{c} für eine Alkoholat-Gruppe mit einem C1- bis C6- Kohlenwasserstoffrest steht.

4. Verfahren gemäß einem der vorhergehenden Ansprüche, **dadurch gekennzeichnet, dass** die Umsetzung in Gegenwart von stetig zugeführtem Sauerstoff in Form eines Gasgemisches, das Sauerstoff in einer Konzentration von 5 bis 30 Vol.-% enthält, durchgeführt wird.

5. Verfahren gemäß einem der vorhergehenden Ansprüche, **dadurch gekennzeichnet, dass** die Umsetzung bei Temperaturen von 0 °C bis 150 °C durchgeführt wird.

6. Verfahren gemäß einem der vorhergehenden Ansprüche, **dadurch gekennzeichnet, dass** die Umsetzung bei Drücken von 2 bar_{abs} bis 0,01 bar_{abs} durchgeführt wird.

7. Verfahren gemäß einem der vorhergehenden Ansprüche, **dadurch gekennzeichnet, dass** die Umsetzung lösemittelfrei durchgeführt wird.

8. Verfahren gemäß einem der Ansprüche 1 bis 5, **dadurch gekennzeichnet, dass** die Umsetzung in zumindest einem der nachgenannten Lösemittel durchgeführt wird: Kohlenwasserstoffe, Ester, Ether, Glycole und Glycol-mono-/diether.

9. Verfahren gemäß einem der vorhergehenden Ansprüche, **dadurch gekennzeichnet, dass** die Carbonsäure Acrylsäure oder Metacrylsäure ist.

10. Verfahren gemäß einem der vorhergehenden Ansprüche, **dadurch gekennzeichnet, dass** das Metall M Aluminium und/oder Zirkonium ist.

11. Verfahren gemäß einem der vorhergehenden Ansprüche, **dadurch gekennzeichnet, dass** die Metallverbindung ein Metallalkoholat ist.

12. Verfahren gemäß einem der vorhergehenden Ansprüche, **dadurch gekennzeichnet, dass** die Umsetzung in Abwesenheit von Wasser (kleiner 100 ppm) erfolgt.

13. Verwendung von Metallsalze herstellbar nach einem der Ansprüche 1 bis 12 aufweisend zumindest eine ungesättigte Carboxylgruppe mit 3 bis 7 Kohlenstoffatomen in der Carboxylgruppe bzw. deren Umsetzungsprodukten als oder in Beschichtungen und in Gummi-Werkstoffen.

14. Verwendung von Metallsalzen herstellbar nach einem der Ansprüche 1 bis 12 als oder in Beschichtungsmaterial(ien) zur Beschichtung von Leder, Glas, Keramiken, Papier, Pappe, Kunststoffe, Metalle und Textilien.

15. Verwendung von Metallsalzen herstellbar nach einem der Ansprüche 1 bis 12 aufweisend zumindest eine ungesättigte Carboxylgruppe mit 3 bis 7 Kohlenstoffatomen in der Carboxylgruppe als Monomer in Polymerisationsprozessen.

16. Verwendung von Metallsalzen herstellbar nach einem der Ansprüche 1 bis 12 aufweisend zumindest eine ungesättigte Carboxylgruppe mit 3 bis 7 Kohlenstoffatomen in der Carboxylgruppe als Additiv in strahlenhärtenden Kleberzusammensetzungen oder Kunststoffzusammensetzungen.

17. Verwendung von Metallsalzen herstellbar nach einem der Ansprüche 1 bis 12 aufweisend zumindest eine ungesättigte Carboxylgruppen mit 3 bis 7 Kohlenstoffatomen in der Carboxylgruppe in Druckfarbenzusammensetzungen.

18. Verwendung von Metallsalzen herstellbar nach einem der Ansprüche 1 bis 12 aufweisend zumindest eine ungesättigte Carboxylgruppe mit 3 bis 7 Kohlenstoffatomen in der Carboxylgruppe bzw. deren Umsetzungsprodukten als Rheologiemodifikator.

19. Verwendung von Metallsalzen herstellbar nach einem der Ansprüche 1 bis 12 aufweisend zumindest eine ungesättigte Carboxylgruppe mit 3 bis 7 Kohlenstoffatomen in der Carboxylgruppe bzw. deren Umsetzungsprodukten als oder in Barriere-Beschichtungen für Folien gegen den Durchtritt von Sauerstoff und/oder Wasser.

20. Verwendung der Metallsalze herstellbar nach einem der Ansprüche 1 bis 12 gemäß zumindest einem der Ansprüche 13 bis 19, **dadurch gekennzeichnet, dass** die eingesetzten Zusammensetzungen enthaltend die Metallsalze weiterhin
■ 1 bis 5 Gew.% Photoinitiatoren, und/oder
■ 0,05 bis 2 Gew.% UV- und/oder Radikal-Stabilisatoren
enthalten.

## Claims

1. A method for the production of metal salts of short-chain, unsaturated carboxylic acids by reacting
- metal alcoholate compounds
- with carboxylic acids of the general formula
CₙH₂ₙ₋₁C(=O)OH
with double bond in 2- or 3-position, wherein n represents 2, 3, 4, 5, or 6,
and/or maleic acid,
- in the presence of oxygen (O₂); oxygen being continuously supplied in such a way that the reaction solution is oxygen saturated to least 50 %, and
said metal salts exhibiting at least one group of the formula
CₙH₂ₙ₋₁C(=O)O- and/or -OC(=O)CH=CHC(=O)O-(H)
and containing the following metals or mixtures thereof
Al, Si, Sn, La, Zr, Cu and/or Zn.

2. The method according to claim 1, **characterized in that** oxygen is continuously supplied in such a way that the reaction solution is oxygen saturated to at least 90 %.

3. The method according to claim 1 or claim 2, **characterized in that** the metal salts correspond to the general formula
M(OOCCₙH₂ₙ₋₁)ₐ(R¹)_{b}
and are made by reacting a linear or branched, unsaturated carboxylic acid of the formula
CₙH₂ₙ₋₁-COOH,
wherein n is 2, 3, 4, 5, or 6, with double bond in 2- or 3-position with a metal compound of the general formula
M(R¹)_{c}
and optionally
H(R¹)
wherein
a is at least 1; b is 0, 1, 2, or 3, and (a+b) and c are independently of each other an integer from 2 to 4;
M represents the metal according to claim 1,
R¹ represents an alcoholate group having a C1 to C6 hydrocarbon radical, R¹ being a saturated, linear or branched alcoholate group producible from an alcohol having at least one -OH group,
or 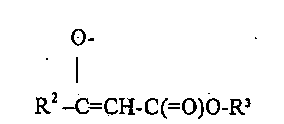 with R² and R³ being -CH₃, -C₂H₅, -C₃H₇ or -C₄H₉, and
wherein n, R¹, R², and R³ may be different for each a, b, and c and at least one R¹ in M(R¹)_{c} represents an alcoholate group having a C1 to C6 hydrocarbon radical.

4. The method according to any one of the previous claims, **characterized in that** the reaction is performed in the presence of continuously supplied oxygen in form of a gaseous mixture that contains oxygen in a concentration of 5 to 30 % by volume.

5. The method according to any one of the previous claims, **characterized in that** the reaction is performed at temperatures of 0 °C to 150 °C.

6. The method according to any one of the previous claims, **characterized in that** the reaction is performed at pressures of 2 bar_{abs} to 0.01 bar_{abs}.

7. The method according to any one of the previous claims, **characterized in that** the reaction is performed solvent-free.

8. The method according to any one of claims 1 to 5, **characterized in that** reaction is performed in at least one of the solvents specified below: hydrocarbons, esters, ethers, glycols, and glycol mono-/diethers.

9. The method according to any one of the previous claims, **characterized in that** the carboxylic acid is acrylic acid or methacrylic acid.

10. The method according to any one of the previous claims, **characterized in that** the metal M is aluminum and/or zirconium.

11. The method according to any one of the previous claims, **characterized in that** the metal compound is a metal alcoholate.

12. The method according to any one of the previous claims, **characterized in that** the reaction takes place in the absence of water (less than 100 ppm).

13. A use of metal salts that are producible according to any one of claims 1 to 12 and having at least one unsaturated carboxyl group with 3 to 7 carbon atoms in the carboxyl group or reaction products thereof as or in coatings and in rubber materials.

14. The use of metal salts that are producible according to any one of claims 1 to 12 as or in coating material(s) for coating leather, glass, ceramics, paper, cardboard, plastics, metals, and textiles.

15. The use of metal salts that are producible according to any one of claims 1 to 12 and having at least one unsaturated carboxyl group with 3 to 7 carbon atoms in the carboxyl group as monomer in polymerization processes.

16. The use of metal salts that are producible according to any one of claims 1 to 12 and having at least one unsaturated carboxyl group with 3 to 7 carbon atoms in the carboxyl group as additive in radiation-curing adhesive compositions or plastic compositions.

17. The use of metal salts that are producible according to any one of claims 1 to 12 and having at least one unsaturated carboxyl group with 3 to 7 carbon atoms in the carboxyl group in printing ink compositions.

18. The use of metal salts that are producible according to any one of claims 1 to 12 and having at least one unsaturated carboxyl group with 3 to 7 carbon atoms in the carboxyl group or reaction products thereof as rheology modifier.

19. The use of metal salts that are producible according to any one of claims 1 to 12 and having at least one unsaturated carboxyl group with 3 to 7 carbon atoms in the carboxyl group or reaction products thereof as or in barrier coatings for foils to prevent the passage of oxygen and/or water.

20. The use of the metal salts that are producible according to any one of claims 1 to 12 in accordance with at least any one of claims 13 to 19, **characterized in that** the compositions used containing said metal salts further contain
■ 1 to 5 % by weight of photoinitiators, and/or
■ 0.05 to 2 % by weight of UV and/or radical stabilizers.

## Revendications

1. Procédé de production de sels métalliques d'acides carboxyliques insaturés à chaînes courtes, par conversion
- de composés d'alcoolates métalliques
- avec des acides carboxyliques de formule générale
CₙH₂ₙ₋₁C(=O)OH
avec la double liaison en position 2 ou 3, dans laquelle n vaut 2, 3, 4, 5 ou 6, et/ou de l'acide maléique,
- en présence d'oxygène (O₂), où l'on amène en continu de l'oxygène de sorte que la solution réactionnelle soit saturée en oxygène à au moins 50 %, et
les sels métalliques présentent au moins un groupe de formule
CₙH₂ₙ₋₁C(=O)O- et/ou -OC(=O)CH=CHC(=O)O-(H)
et les métaux suivants ou leurs mélanges
Al, Si, Sn, La, Zr, Cu et/ou Zn.

2. Procédé selon la revendication 1, **caractérisé en ce que** l'on amène en continu de l'oxygène de sorte que la solution réactionnelle soit saturée en oxygène à au moins 90%.

3. Procédé selon la revendication 1 ou 2, **caractérisé en ce que** les sels métalliques correspondent à la formule générale
M(OOCCₙH₂ₙ₋₁)ₐ(R¹)_{b}
et, par conversion d'un acide carboxylique insaturé, linéaire ou ramifié, de formule
CₙH₂ₙ₋₁-COOH,
dans laquelle n vaut 2, 3, 4, 5 ou 6, avec la double liaison en position 2 ou 3, avec un composé métallique de formule générale
M(R¹)_{c}
et le cas échéant
H(R¹)
dans laquelle
a vaut au moins 1 ; b vaut 0, 1, 2 ou 3 et (a+b) et c représentent, indépendamment les uns des autres, un nombre entier de 2 à 4,
M représente le métal selon la revendication 1,
R¹ représente un groupe alcoolate ayant un radical hydrocarbure en C₁ à C₆, où R¹ est un groupe alcoolate saturé, linéaire ou ramifié, pouvant être produit à partir d'un alcool avec au moins un groupe -OH,
ou 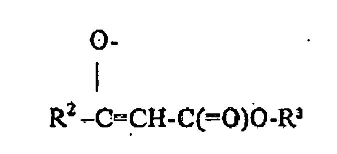 avec R² ou R³ égal à -CH₃, -C₂H₅, -C₃H₇ ou -C₄H₉, et
où n, R¹, R² et R³ peuvent être différents pour chaque a, b ou c et où au moins un R¹ dans M(R¹)_{c} représente un groupe alcoolate avec un radical hydrocarbure en C₁ à C₆.

4. Procédé selon l'une des revendications précédentes, **caractérisé en ce que** la conversion s'effectue en présence d'oxygène amené en continu sous la forme d'un mélange gazeux, qui contient de l'oxygène en une concentration de 5 à 30 % en volume.

5. Procédé selon l'une des revendications précédentes, **caractérisé en ce que** la conversion s'effectue à des températures allant de 0°C à 150°C.

6. Procédé selon l'une des revendications précédentes, **caractérisé en ce que** la conversion s'effectue à des pressions de 2 bar_{abs} à 0,01 bar_{abs}.

7. Procédé selon l'une des revendications précédentes, **caractérisé en ce que** la conversion s'effectue sans solvant.

8. Procédé selon l'une des revendications 1 à 5, **caractérisé en ce que** la conversion s'effectue dans au moins un des solvants cités ci-après : hydrocarbures, esters, éthers, glycols et mono-/diéthers de glycol.

9. Procédé selon l'une des revendications précédentes, **caractérisé en ce que** l'acide carboxylique est l'acide acrylique ou l'acide méthacrylique.

10. Procédé selon l'une des revendications précédentes, **caractérisé en ce que** le métal M est l'aluminium et/ou le zirconium.

11. Procédé selon l'une des revendications précédentes, **caractérisé en ce que** le composé métallique est un alcoolate métallique.

12. Procédé selon l'une des revendications précédentes, **caractérisé en ce que** la conversion s'effectue en l'absence d'eau (taux inférieur à 100 ppm).

13. Utilisation de sels métalliques pouvant être produits selon l'une des revendications 1 à 12, présentant au moins un groupe carboxyle insaturé avec 3 à 7 atomes de carbone dans le groupe carboxyle ou ses produits de conversion en tant que ou dans des revêtements et dans des matériaux caoutchouteux.

14. Utilisation de sels métalliques pouvant être produits selon l'une des revendications 1 à 12 en tant que ou dans des matériaux de revêtement pour le revêtement du cuir, du verre, des céramiques, du papier, du carton, des matières plastiques, des métaux et des textiles.

15. Utilisation de sels métalliques pouvant être produits selon l'une des revendications 1 à 12, présentant au moins un groupe carboxyle insaturé avec 3 à 7 atomes de carbone dans le groupe carboxyle comme monomère dans les procédés de polymérisation.

16. Utilisation de sels métalliques pouvant être produits selon l'une des revendications 1 à 12, présentant au moins un groupe carboxyle insaturé avec 3 à 7 atomes de carbone dans le groupe carboxyle comme additif dans des compositions adhésives ou compositions plastiques durcissables aux rayonnements.

17. Utilisation de sels métalliques pouvant être produits selon l'une des revendications 1 à 12, présentant au moins un groupe carboxyle insaturé avec 3 à 7 atomes de carbone dans le groupe carboxyle dans des compositions d'encre d'impression.

18. Utilisation de sels métalliques pouvant être produits selon l'une des revendications 1 à 12, présentant au moins un groupe carboxyle insaturé avec 3 à 7 atomes de carbone dans le groupe carboxyle ou ses produits de conversion en tant que modificateurs de rhéologie.

19. Utilisation de sels métalliques pouvant être produits selon l'une des revendications 1 à 12, présentant au moins un groupe carboxyle insaturé avec 3 à 7 atomes de carbone dans le groupe carboxyle ou ses produits de conversion en tant que ou dans des revêtements barrières pour des films contre la pénétration de l'oxygène et/ou de l'eau.

20. Utilisation de sels métalliques pouvant être produits selon l'une des revendications 1 à 12 d'après au moins une des revendications 13 à 19, **caractérisée en ce que** les compositions mises en oeuvre contenant des sels métalliques renferment en outre :
■ 1 à 5 % en poids de photoinitiateurs, et/ou
■ 0,05 à 2 % en poids de stabilisants UV et/ou radicalaires.
